# EUROPEAN PATENT APPLICATION

(11) **EP 3 381 352 A1**
(43) Date of publication of application: **03.10.2018**
(21) Application number: 17163533.7
(22) Date of filing: 29.03.2017
(51) Int. Cl.: A61B 5/00, A61B 5/0215, A61B 5/03

(54) **INVASIVE INSTRUMENT WITH SENSOR FUNCTIONALITY**

(71) Applicant: Koninklijke Philips N.V., 5656 AE Eindhoven (NL)
(72) Inventor: VAN RENS, Antonia Cornelia, 5656 AE Eindhoven (NL)
(74) Representative: de Haan, Poul Erik

(57) **Abstract**

An invasive instrument (1) is disclosed comprising an instrument tip including a sensor chip (10) comprising a sensor (20) adapted to produce a modulated sensor signal having a signal frequency and a non-volatile memory (30) responsive to said sensor signal for storing data relating to said sensor; a first supply wire (11) for connecting the sensor chip to ground and a second supply wire (13) for connecting the sensor chip to a power supply (61) for providing a supply voltage to the sensor chip. The device further comprises a circuitry arrangement arranged to combine said sensor signal and said output into a combined signal and superimpose said combined signal as a modulation over the supply voltage such that simultaneous data communication of the sensor and memory data can be provided using two interface wires only. A system including such an invasive instrument and a method of operating such a system are also disclosed.

## Description

### FIELD OF THE INVENTION

The present invention relates to an invasive instrument comprising an instrument tip including a sensor chip comprising a sensor adapted to produce a sensor signal having a defined frequency and a non-volatile memory for storing data relating to said sensor.

The present invention further relates to a system including such an invasive instrument and a method of operating such a system.

### BACKGROUND OF THE INVENTION

Invasive instruments are commonly deployed in medicine to investigate and/or treat anomalies within a patient's body. Examples of such devices include catheters and guidewires but are not limited thereto. It is desirable to extend such invasive instruments with one or more sensors such as for example pressure sensors, which may assist in the correct application of the invasive instrument within the patient's body, e.g. to avoid an insufficient or excessive amount of pressure being applied to an anomaly by monitoring of the sensor data, and/or to enhance the diagnostic capabilities of the invasive instrument, as the sensor data may supplement the primary data of intermediate diagnostic relevance, such as ultrasound data in case the invasive instrument is fitted with one or more ultrasound transducers, optical data in case the invasive instrument is fitted with one or more image sensors or cameras, and so on.

Where such sensors are included in an invasive instrument, the device typically also requires a memory module to store at least one of sensor calibration data, sensor ID code and data gathered during a procedure with the invasive instrument, which memory module typically is read out through a wired connection between the memory module and a reader. For this reason, such a memory module often is located in a connector, e.g. a plug, for the invasive instrument, as such a connector does not suffer from the same size constraints as the device itself, such that there is more space in the connector to house the memory module, thereby facilitating the use of standard size industrial components.

A common configuration used in such an arrangement is that the data communication between a control module on the one hand and the sensor and memory module of the invasive instrument on the other hand is performed using a 1-Wire bus as developed by the Dallas Semiconductor Corporation, in which the supply wires for the memory module are shared with the sensor. However, this has the disadvantage that the memory module and the sensor need to be paired throughout the assembly process in order to facilitate the storage of calibration data in the memory module gathered during this process. Such pairing is prone to mistakes, which reduces the manufacturing yield of such invasive instruments. Given that such devices typically are rather expensive, such yield losses are highly undesirable.

For this reason, a commonly used alternative approach is to incorporate the memory module on the sensor chip at the tip of the invasive instrument. As explained above, this has its own challenges because such instrument tips are rather constrained in terms of their allowable dimensions (form factors) as they must be small enough to be inserted into the patient's body and to reach target locations therein. For this reason, there are clear design constraints to the number of interface wires between the instrument tip and the connector of the invasive instrument.

US 9,050,001 B2 discloses a monitoring system including a reading device electrically connected to a probe via a wired interface. The probe has a physiological sensor/transducer configured as a Wheatstone resistive bridge balancing circuit. Integrated within the housing of the probe to prohibit separation during use by a user is a memory device arranged in parallel with the sensor. Communication between the reading device and the probe occurs via a wired interface utilizing a same number of electrical wires between the reading device and the Wheatstone as would be required without the memory device. Control circuitry selects between one of two modes for accessing either data detected by the sensor or the memory device. This system utilizes a 4-wire interface between the reading device and the probe. However, there exists a need to further reduce the number of interface wires, for example to facilitate a more compact invasive instrument.

### SUMMARY OF THE INVENTION

The present invention seeks to provide an invasive instrument comprising an instrument tip including a sensor chip comprising a sensor adapted to produce a sensor signal at a certain frequency and a non-volatile memory for storing data relating to said sensor that requires fewer interface wires to extract sensor and memory data from the device.

The present invention further seeks to provide a system including such an invasive instrument.

The present invention yet further seeks to provide a method of operating such a system.

According to an aspect, there is provided an invasive instrument comprising an instrument tip including a sensor chip comprising a sensor adapted to produce a modulated sensor signal having a signal frequency and a non-volatile memory for storing data relating to said sensor and adapted to generate a memory output signal including at least a portion of said data in response to said sensor signal; a first supply wire for connecting the sensor chip to ground; a second supply wire for connecting the sensor chip to a power supply for providing a supply voltage to the sensor chip; and a circuitry arrangement arranged to combine said sensor signal and said memory output signal into a combined signal and superimpose said combined signal as a modulation over the supply voltage.

The invasive instrument according to embodiments of the present invention thus provides the ability to simultaneously deliver sensor data as well as memory module data over one of the pair of supply wires whilst at the same time reducing the required number of interface wires to facilitate data communication between the sensor and memory modules on the one hand and a control module of the invasive instrument on the other hand. In an embodiment, the circuitry arrangement comprises a multiplier having an output coupled to the second supply wire and arranged to receive the sensor signal as a first input and the memory output signal as a second input and being arranged to produce the combined signal as a multiplication result of said sensor signal and said memory output signal.

The modulation of the sensor signal defines the data produced by the sensor. Such modulation may be provided in any suitable manner. Preferably, the modulated sensor signal has a variable signal frequency, said variation defining the modulation, i.e. is frequency-modulated, although alternatively the modulated sensor signal may have a fixed signal frequency which is modulated with a digital modulation method to define the sensor data. The chosen modulation method for example includes an embedded clock signal that can control the data extraction from the memory and can be detected in the system to be able to read the data.

Preferably, the non-volatile memory is a one-time programmable memory such that data critical to the correct operation and interpretation of the sensor data, e.g. sensor calibration data, stored in the non-volatile memory is protected from being accidentally overwritten during use of the invasive instrument, thereby protecting the device from being rendered inoperable by such accidental overwriting.

The invasive instrument may further comprise a filter positioned along the second supply wire in between the multiplier and the power supply, said filter being arranged to remove said modulation from the supply voltage provided to the sensor chip in order to ensure that the sensor chip is provided with a stable supply voltage.

The non-volatile memory preferably comprises a data shift register coupled to the multiplexer and comprising a plurality of register elements for storing the data relating to said sensor; and an address shift register responsive to the sensor signal and containing a plurality of address pointers to respective register elements of the data shift register. Such a non-volatile memory benefits from being implicitly addressed by the sensor signal, with the memory cycling through its data shift register elements at the frequency of the sensor signal.

In an embodiment, the data shift register may have been programmed with the data relating to said sensor in an encoded format such as to facilitate distinction between the sensor signal and the memory data upon interpreting the modulation of the supply voltage, e.g. by a readout module of control module of the invasive instrument. Such an encoding format preferably is a DC-free encoding format such as 8b/10b coding in order to protect the supply voltage provided to the sensor chip from DC fluctuations caused by DC offsets in the supply voltage modulation. The coding format 8b/10b coding is particularly suitable as it uses so-called unique "K-codes" to achieve a form of frame-synchronization, which is utilized by the present invention for memory address recognition, thus obviating the need for explicit memory address programming.

The data shift register may comprise a hard-coded region for storing sensor identification information, such that the sensor identification information does not require programming into the data shift register, e.g. during its calibration. The hard-coded region also includes identification information defining a start point of a programmable part of the data shift register.

The non-volatile memory may be programmed autonomously by the sensor chip, in which case no additional wiring between a control module and the invasive instrument is required to facilitate reception of the programming data by the non-volatile memory. Alternatively, the invasive instrument may further comprise a probe pad or wire bond coupled to the data shift register to facilitate upload of sensor-related data during integration of the invasive instrument, i.e. whilst the probe pad is still accessible during the integration process, or the invasive instrument may further comprise a programming interface wire coupled to the data shift register to facilitate upload of such data upon completion of the integration of the device (in which case the probe pads of the sensor chip may no longer be accessible).

In example embodiments, the invasive instrument is a guidewire or a catheter. Such devices particularly require small instrument tips in order to enable their use as intended, and therefore particularly benefit from the teachings of the present invention, which as explained above assists in reducing the form factor of the instrument tip due to the fact that simultaneous communication of data from the sensor and the non-volatile memory requires to supply wires only. The invasive instrument may be designed for diagnostic purposes and/or for surgical purposes. Typically, the invasive instrument is designed for invasive procedures on mammals, most notably human patients.

In example embodiments, the sensor is a pressure sensor, although other types of sensors maybe contemplated as well. Such pressure sensors maybe used in procedures such as a so-called FFR procedure (fractional flow reserve), during which blood pressure is measured proximal and distal to a stenosed region (lesion) in a (coronary) artery. The ratio of the two pressures reflects the impact of the lesion and provides decisive input to the decision on whether a stent is to inserted in the artery, as well as where this stent should be placed.

According to another aspect, there is provided a system comprising the invasive instrument of any of the herein described embodiments; and a control module including a power supply having a ground terminal for connecting to the first supply wire and a supply voltage terminal for connecting to the second supply wire; and a readout module adapted to extract the sensor signal and the memory output signal from the modulated supply voltage. Such a system benefits from the ability to simultaneously read out data from the sensor and the non-volatile memory, whilst at the same time requiring only two supply wires in order to facilitate such simultaneous readout.

According to yet another aspect, there is provided a method of operating such a system the method comprising connecting the invasive instrument to the control module such that the ground terminal is connected to the first supply wire and the supply voltage terminal is connected to the second supply wire; controlling the invasive instrument by supplying a supply voltage with the power supply to the second supply wire; receiving a supply voltage modulated with a combined signal of said sensor signal and said memory output signal over the second supply wire; and extracting the sensor signal and the memory output from the modulated supply voltage with the readout module. With such a method, sensor data and non-volatile memory data can be read out simultaneously from the invasive instrument using only two supply wires, thereby providing an efficient method of obtaining such data in terms of readout time and size of the invasive instrument.

The method may further comprise programming the non-volatile memory with the data relating to said sensor in an encoded form such that the data from the memory can be more readily distinguished from the sensor data when retrieved from the modulated supply voltage.

### BRIEF DESCRIPTION OF THE DRAWINGS

Embodiments of the invention are described in more detail and by way of nonlimiting examples with reference to the accompanying drawings, wherein:
FIG. 1 schematically depicts an aspect of an invasive diagnostic tool according to an embodiment;
FIG. 2 schematically depicts an example set of data signals generated with the invasive diagnostic tool according to embodiments of the present invention;
FIG. 3 schematically depicts another example set of data signals generated with the invasive diagnostic tool according to embodiments of the present invention;
FIG. 4 schematically depicts an aspect of an invasive diagnostic tool according to another embodiment;
FIG. 5 schematically depicts an aspect of an invasive diagnostic tool according to yet another embodiment; and
FIG. 6 is a flowchart of an example embodiment of a method of operating a system including the invasive diagnostic tool according to embodiments of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

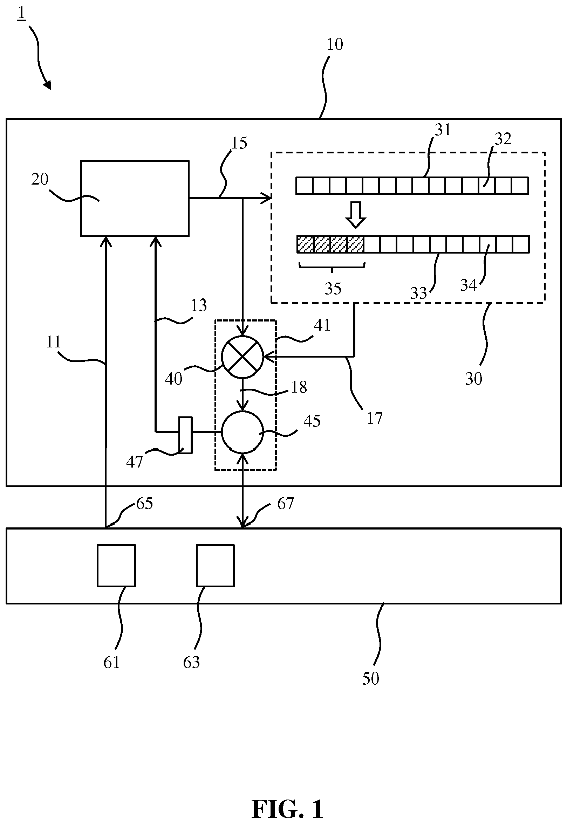

FIG. 1 schematically depicts a system for invasive procedures according to an embodiment of the present invention. The system comprises an invasive instrument 1 such as a catheter or a guidewire, which device 1 may be coupled to a control module 50 for controlling operation of the invasive instrument 1, e.g. when inserted in a patient. Such a coupling may be achieved in any suitable manner. For example, the coupling may be a plug- and-socket type coupling in which the invasive instrument 1 comprises a plug, e.g. at the end of a connector cable, which plug mates with a socket of the control module 50. Other suitable coupling mechanisms will be immediately apparent to the skilled person.

The invasive instrument 1 comprises a sensor chip 10 comprising a sensor 20. The sensor chip 10 may be an application specific integrated circuit (ASIC), which may be manufactured using any suitable semiconductor technology. The sensor 20 may be any type of sensor suitable to extend the functionality of the invasive instrument 1. In specific embodiments, the sensor 20 is a pressure sensor adapted to sense a pressure in tissue in which the invasive instrument 1 comes into contact with. The sensor chip 10 typically is located at the tip of the invasive instrument 1. In the context of the present application, the instrument tip is defined as the part of the invasive instrument 1 that is diagnostically relevant, i.e. the part of the device used to collect data based on which a clinician may base a diagnostic finding.

The sensor chip 10 is powered by a power supply module 61 of the control module 50 through a first supply wire 11 connecting the sensor chip 10 to a ground terminal 65 of the control module 50 and a second supply wire 13 over which the power supply module 61 provides the sensor chip 10 with a supply voltage through a supply terminal 67 of the control module 50. The second supply wire 13 is utilized by the sensor 20 to provide a readout module 63 of the control module 50 with the sensor signals generated by the sensor 20, which sensor signals are added by the sensor 20 to the supply voltage as a modulation of the supply voltage through sensor output connection 15 of the sensor chip 10 in an example embodiment, although it should be understood that the sensor 20 may produce any suitable output signal including the sensor data as a modulation. Such a modulation may be achieved in a number of manners. In a preferred embodiment, the modulation is a frequency modulation. In the context of the present application, such a frequency modulation is a modulation in which the (pressure) sensor signal is represented by a frequency f with f changing as a function of the pressure sensed by the pressure sensor 20. Such a frequency-modulated output signal is attractive as such a "quasi-digital" signal is robust to interference across the supply wires 11, 13.

Alternatively, the sensor signal may be modulated as a digital code superimposed on a fixed clock frequency of the sensor 20, which modulation thus implements a digital modulation method with an embedded clock signal. The embedded clock signal is then used to decode the data.

The first supply wire 11 and the second supply wire 13 typically define an interface between the sensor chip 10 and the control module 50 and are not to be understood to form part of the sensor chip 10 in at least some embodiments of the present invention.

The sensor chip 10 further comprises a non-volatile memory 30 in which data relating to the sensor 20 can be stored. Such data for example includes an identification code for the sensor 20, calibration data for the sensor 20 and/or data collected during a (diagnostic) procedure performed with the invasive instrument 1. Any suitable non-volatile memory 30 may be used for this purpose, e.g. a PROM, EEPROM, and so on. In case of the storage of identification information and calibration data for the sensor 20, the non-volatile memory 30 preferably is a one-time programmable memory in order to prevent the accidental overwriting of such data, which would render the sensor 20 unidentifiable or otherwise unusable. The non-volatile memory 30 typically is dimensioned such that it meets the sizing requirements that are applicable to the tip of the invasive instrument 1 as will be readily understood by the skilled person.

The non-volatile memory 30 in a preferred embodiment comprises a data shift register 33 comprising a plurality of shift register elements or cells 34 in which the data relating to the sensor 20 can be stored. The non-volatile memory 30 further comprises an address shift register 31 comprising a plurality of shift register elements or cells 32 in which address pointers to corresponding shift register elements 34 of the data shift register 33 are stored. The address shift register 31 is responsive to the frequency of the sensor signal produced by the sensor 20, e.g. to its embedded clock signal, such that with each period of the sensor signal of the sensor 20, the address shift register 31 shifts to the next address shift register element 32, thereby selecting a next data shift register element 34 as indicated by the address pointer stored in the addressed (selected) address shift register element 32. As will be understood from the foregoing, this may be a variable frequency signal in case of a frequency-modulated sensor signal, or a fixed frequency signal in case of a sensor signal modulated with a digital modulation method.

The selected data shift register element 34 outputs the data stored therein on memory output 17. In an embodiment, the data shift register 33 comprises a hard-coded region 35 consisting of one or more data shift register elements 34 of the data shift register 33, which hard-coded region 35 may be used to store an identification code of the sensor 20 or other information about the sensor 20, and may define the start of the part of the data shift register 33 that can be programmed as previously explained.

In an example embodiment, the invasive instrument 1 further comprises a circuit arrangement 41 including a multiplier circuit element 40, which receives the modulated sensor output signal from sensor output 15 as a first input and the memory data signal from non-volatile memory output 17 as a second input, and which multiplies the (frequency) modulated output signal of the sensor 20 with the data from the non-volatile memory 30 provided over the memory output 17 in order to further modulate the sensor signal and produce this further modulated voltage supply signal on its output 18.

As a result, the output 18 of the multiplier 40 comprises a combined signal which conveys two data streams. The first is the output signal from the sensor, which in this example is expressed by the frequency of the signal at the output 18, and the second is a bit sequence from the non-volatile memory. Thus, in this example there is a sequence of pulses which form a bit sequence, but the bit frequency is variable and that variable frequency encodes the sensor information. The signal thus is designed to enable the bit frequency to be determined, regardless of the bit pattern encoded from the non-volatile memory 30.

In essence, there are two orthogonal modulation schemes, namely two independent modulations which may be de-modulated independently. The example above comprises a frequency modulation followed by the formation of a bit sequence. However, any pair of modulation techniques may be used. The demodulation needs to be able to extract a clock signal to enable interpretation of the bit sequence from the memory, as well as extracting the sensor signal. The sensor signal may be provided as an analog modulation or a digital modulation. The possible options for the double modulation will be apparent to those skilled in the art of signal processing.

The circuit arrangement 41 including the multiplier circuit element 40 preferably forms part of the sensor chip 10, although it should be understood that the multiplier circuit element 40 alternatively may be provided separate to the sensor chip 10, e.g. on a separate chip position in between the sensor chip 10 and the control module 50. In order to facilitate the extraction of the modulated sensor signal from the memory data, i.e. prevent interference between the modulated sensor signal and the memory data, the memory data preferably is stored in the data shift register 33 in an encoded format using any suitable form of line coding. Such an encoding format preferably is a DC-free encoding format, in which over a defined period of time the number of binary 1s and 0s are equal or does not differ by more than one in order to achieve DC-balance on the second supply wire 13 between the sensor chip 10 and the control module 50, i.e. the power supply 61. A particularly suitable encoding format is 8b/10b encoding, as it is well-known per se that such a line code can achieve DC-balance and bounded disparity whilst still providing enough state changes (data transitions) to allow reasonable clock recovery, reserved codes for synchronization, data-stitching and error correction, and so on. Such line codes, and in particular 8b/10b encoding, allow for the frequency-modulated sensor signal and the memory data to be combined by the multiplier circuit element 40 without influencing each other.

A further advantage of 8b/10b encoding is that it contains so-called K-codes, which are unique codes that may be utilized to achieve a form of frame-synchronization, which may be utilized for memory address recognition The thus further modulated supply voltage on the second supply wire 13 may be processed by the readout module 63 of the control module 50 to extract the sensor signal and the memory data signal from the further modulated supply voltage. As the processing of frequency-modulated and line coded signals is well-known per se, the function of the readout module 63 is not further explained for the sake of brevity only.

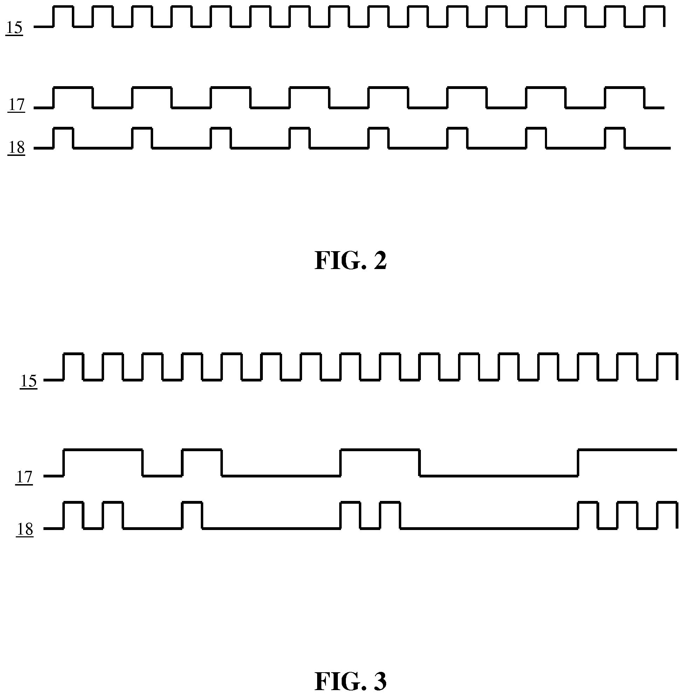

FIG. 2 and FIG. 3 schematically depict examples of the functionality of the multiplier circuit element 40, in particular how the multiplier circuit element 40 combines the modulated voltage supply signal, i.e. the sensor signal, from sensor output 15 with the memory data signal from memory output 17 into the further modulated voltage supply signal on the output 18 of the multiplier circuit element 40.

The signal at the multiplier output 18 is superposed over the supply voltage on the supply terminal 67 by an adder 45 of the circuitry arrangement 41 in such a way that the superposed signal may be extracted in the control module to enable the demodulation of the two data streams (the sensor signal and the memory data). The adder 45 is arranged between the multiplier 40 and the control module 50 (i.e. the power supply 61). The supply voltage is for example a DC voltage, on which a smaller magnitude AC modulation signal is superposed. This AC signal may have a fixed or variable frequency as described above.

The sensor 20 may be designed such that the sensor is robust against fluctuations on the power supply, e.g. fluctuations caused by modulations imparted upon the power supply signal, as such modulations typically are at least an order of magnitude smaller than the power supply signal itself. However, where the sensor 20 may lack such robustness, in order to ensure that the various components of the sensor chip 10 such as the sensor 20 are provided with a supply voltage without the aforementioned modulation, the invasive instrument 1 optionally further comprises a filter 47 located for example in between the adder 45 and the sensor 20 that subtracts the further modulation produced by the multiplier circuit element 40 from the supply voltage to be provided to the sensor 20. As will be readily understood by the skilled person, this ensures that the supply voltage reaching the sensor 20 does not already exhibit any modulation, which could interfere with the modulation imparted onto the supply voltage by the sensor 20. The filter 47 preferably is located on the sensor chip 10 although in alternative embodiments the filter 47 is separate to the sensor chip 10, e.g. located on a separate chip in between the sensor chip 10 and the control module 50.

At this point it is noted that in the above embodiments, the circuitry arrangement 41 for creating the superposition of the combined modulated signal onto the supply voltage comprises the multiplier circuit 40 and the adder circuit 45 by way of nonlimiting example. Any other suitable circuitry arrangement for the generation of such a combined signal and its superposition over the supply voltage may be used, as will be apparent to the person skilled in the art of signal processing.

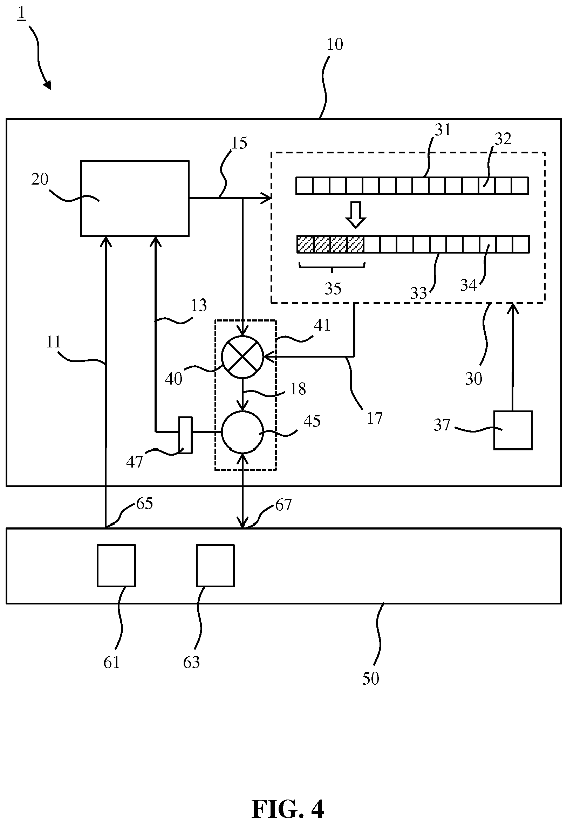

In FIG. 1, the sensor 20 maybe further arranged to self-program the data shift register 33 of the non-volatile memory 30 in order to program the non-volatile memory 30 with the data relating to the sensor 20, e.g. its calibration data or data acquired during an invasive procedure with the invasive instrument 1. This may be achieved in any suitable manner, e.g. through appropriate wiring of the sensor chip 10 as will be readily understood by the skilled person. However, in an alternative embodiment schematically depicted in FIG. 4, the sensor chip 10 contains a programming module 37 coupled to the non-volatile memory 30 adapted to program the data shift register 33 with the data relating to the sensor 20. Such a programming module 37 for example may be activated through a dedicated bond pad of the sensor chip 10, e.g. a probe pad or wire bond pad, prior to integration of the sensor chip 10 into the invasive instrument 1 (such that this dedicated bond pad of the sensor chip 10 is still accessible). Alternatively, the programming module 37 maybe omitted and the non-volatile memory 30 may be programmed directly through such a dedicated bond pad prior to integration of the sensor chip 10 into the invasive instrument 1.

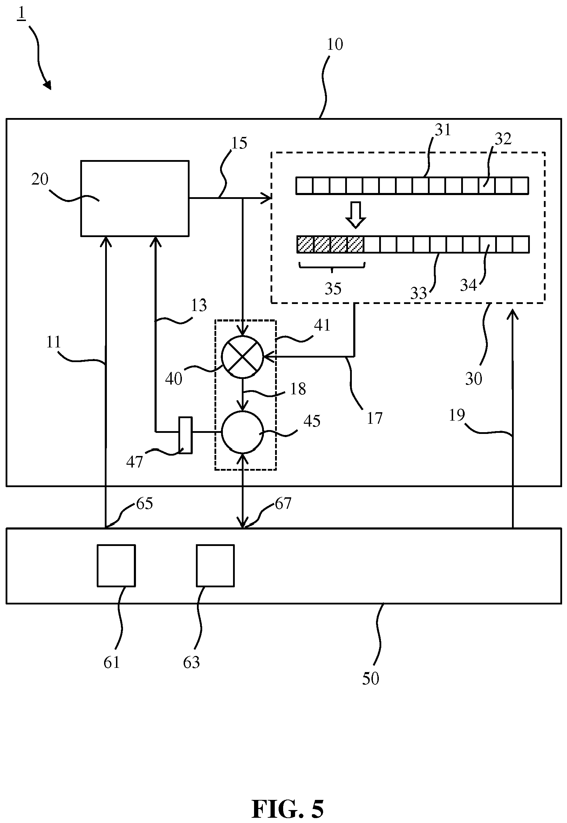

In a scenario where it is desirable that the programming of the non-volatile memory 30 with the data relating to the sensor 20 is performed after such integration of the sensor chip 10 in the invasive instrument 1, the interface between the sensor chip 10 and the control module 50 may comprise an additional interface wire 19 as schematically depicted in FIG. 5 through which the non-volatile memory 30 may be programmed. This may be desirable in terms of facilitating calibration of the sensor 20 upon completion of the integration of the sensor chip 10 within the invasive instrument 1, which may improve the accuracy of the calibration data, but comes at the expense of an additional interface wire between the sensor chip 10 and the control module 50, i.e. in addition to the two-wire implementation consisting of interface wires 11 and 13 as previously described.

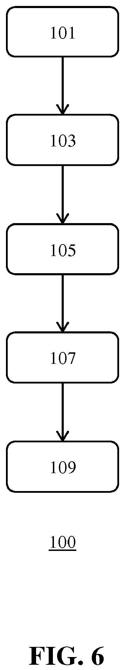

A flowchart of a typical method 100 of operating the embodiments of the system including the invasive instrument 1 and the control module 50 is depicted in FIG. 6. In operation 101, the non-volatile memory 30 is programmed with the data relating to the sensor 20, such as its calibration data, preferably in an encoded format as previously explained. Such programming may further include programming identification information pertaining to the sensor 20 into the non-volatile memory 30, although as previously explained such identification information preferably is hard-coded into a region 35 of the data shift register 33 of the non-volatile memory 30. It is furthermore preferred that operation 101 is only performed once, e.g. in case of a one-time programmable memory 30 to prevent overwriting of the data stored therein. The programming operation 101 may be performed in accordance with any of the example programming options described above.

In operation 103, the invasive instrument 1 is connected to the control module 50 for the performance of an invasive procedure, e.g. an invasive surgical procedure and/or an invasive diagnostic procedure, such that the ground terminal 65 of the control module 50 is connected to the first supply wire 11 interfacing the control module 50 to the sensor chip 10 and the supply voltage terminal 67 is connected to the second supply wire 13 interfacing the control module 50 to the sensor chip 10. Such a connection may be established in any suitable manner, for example by insertion of a plug attached to the invasive instrument 1 into a matching socket of the control module 50 as will be readily understood by the skilled person.

Upon establishing this connection, the invasive instrument 1 may be controlled in operation 105 by supplying a supply voltage with the power supply module 61 to the second supply wire 13 through the supply voltage terminal 67, which supply voltage may be modulated by the sensor 20 and the circuit arrangement including the multiplexer 40 to superimpose the modulation of the combined sensor data and memory data over the supply voltage as previously explained, which modulated supply voltage is received over the second supply wire 13 by the readout module 63 in operation 107, upon which the readout module 63 extracts the sensor signal produced by the sensor 20 and the data from the non-volatile memory 30 from this further modulated supply voltage in operation 109.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An invasive instrument (1) comprising:
an instrument tip including a sensor chip (10) comprising a sensor (20) adapted to produce a modulated sensor signal having a signal frequency and a non-volatile memory (30) for storing data relating to said sensor and adapted to generate a memory output signal including at least a portion of said data in response to said sensor signal;
a first supply wire (11) for connecting the sensor chip to ground;
a second supply wire (13) for connecting the sensor chip to a power supply (61) for providing a supply voltage to the sensor chip; and
a circuitry arrangement (41) arranged to combine said sensor signal and said memory output signal into a combined signal and superimpose said combined signal as a modulation over the supply voltage.

2. The invasive instrument (1) of claim 1, wherein the circuitry arrangement (41) comprises a multiplier (40) having an output (18) coupled to the second supply wire and arranged to receive the sensor signal as a first input and the memory output signal as a second input and being arranged to produce the combined signal as a multiplication result of said sensor signal and said memory output signal.

3. The invasive instrument (1) of claim 1 or 2, wherein the modulated sensor signal has a variable signal frequency, said variation defining the modulation, or wherein the modulated sensor signal has a fixed signal frequency and comprises a digital modulated signal with an embedded clock signal.

4. The invasive instrument (1) of any of claims 1-3, wherein the non-volatile memory (30) is a one-time programmable memory.

5. The invasive instrument (1) of any of claims 1-4, further comprising a filter (47) between the power supply (61) and the sensor (20), said filter being arranged to remove the modulation from the supply voltage provided to the sensor.

6. The invasive instrument (1) of any of claims 1-5, wherein the non-volatile memory (30) comprises:
a data shift register (33) coupled to the multiplier (40) and comprising a plurality of register elements (34) for storing the data relating to said sensor (20); and
an address shift register (31) responsive to the sensor signal and containing a plurality of address pointers (32) to respective register elements of the data shift register.

7. The invasive instrument (1) of claim 6, wherein the data shift register (33) is programmed with the data relating to said sensor in an encoded format.

8. The invasive instrument (1) of claim 7, wherein the encoding format is DC-free encoding, preferably 8b/10b coding.

9. The invasive instrument (1) of any of claims 5-8, wherein the data shift register (33) comprises a hard-coded region (35) storing sensor identification information and identification information defining a start point of a programmable part of the data shift register.

10. The invasive instrument (1) of any of claims 6-9, further comprising a probe pad or wire bond pad coupled to the data shift register (33) or a programming wire (19) coupled to the data shift register (33).

11. The invasive instrument (1) of any of claims 1-10, wherein the invasive instrument is a guidewire or a catheter.

12. The invasive instrument (1) of any of claims 1-11, wherein the sensor (20) is a pressure sensor.

13. A system comprising:
the invasive instrument (1) of any of claims 1-12; and
a control module (50) including:
a power supply module (61) having a ground terminal (65) for connecting to the first supply wire (11) and a supply voltage terminal (67) for connecting to the second supply wire (13); and
a readout module (63) adapted to extract the sensor signal and the memory output signal from the modulation over the supply voltage.

14. A method (100) of operating the system of claim 13, the method comprising:
connecting (103) the invasive instrument (1) to the control module (50) such that the ground terminal (65) is connected to the first supply wire (11) and the supply voltage terminal (67) is connected to the second supply wire (13);
controlling (105) the invasive instrument by supplying a supply voltage with the power supply module (61) to the second supply wire;
receiving (107) a supply voltage modulated with a combined signal of said modulated sensor signal and said memory output signal over the second supply wire; and
extracting (109) the sensor signal and the memory output signal from the modulated supply voltage with the readout module.

15. The method (100) of claim 14, further comprising programming (101) the non-volatile memory (30) with the data relating to said sensor (20) in an encoded format.
